# FASCICULE DE BREVET EUROPEEN

(11) **EP 1 531 771 B1**
(45) Date de publication et mention de la délivrance du brevet: **28.10.2009**
(21) Numéro de dépôt: 03750781.1
(22) Date de dépôt: 18.06.2003
(51) Int. Cl.: A61F 13/08, A61H 23/04

(54) **DISPOSITIF POUR APPLIQUER UNE COMPRESSION CONTROLEE ET MODULABLE SUR UN MEMBRE**
VORRICHTUNG ZUM AUFBRINGEN VON KONTROLLIERTER UND ANPASSBARER KOMPRESSION AUF EINE GLIEDMASSE
DEVICE FOR APPLYING CONTROLLED AND ADJUSTABLE COMPRESSION ON A LIMB

(30) Priorité: 19.06.2002 FR 0207520
(43) Date de publication de la demande: 25.05.2005
(73) Titulaire: Laboratoires Innothera S.A.S., 94110 Arceuil (FR)
(72) Inventeur: OUCHENE, Amina, F-94700 Maisons-Alfort (FR); COUNORD, Jean-Louis, F-92500 Rueil-Malmaison (FR)
(74) Mandataire: Dupuis-Latour, Dominique
(86) Numéro de dépôt international: PCT/FR2003/001850
(87) Numéro de publication internationale: WO 2004/000183

(56) Documents cités:
- WO-A-02/19955
- FR-A- 2 616 064
- US-A- 2 747 570
- US-A- 3 826 249
- US-A- 3 908 642
- US-A- 4 206 751

## Description

L'invention concerne un dispositif pour appliquer une compression contrôlée et modulable sur un membre.

L'invention sera principalement décrite dans le cadre de la compression du membre inférieur, qu'il s'agisse d'une compression du mollet, c'est-à-dire de la partie comprise entre le genou et la cheville, ou de la compression de la jambe entière, de la cheville jusqu'au haut de la cuisse.

Cette application n'est cependant pas limitative, et l'invention pourra aussi bien être mise en oeuvre pour appliquer une compression contrôlée et modulable sur un membre supérieur, par exemple pour le traitement du lymphoedème du bras ou de pathologies similaires, chaque fois qu'il peut être opportun d'envelopper le membre d'un pansement à compression réglable.

Le but premier de l'invention est de disposer d'un appareil d'étude capable de reproduire les effets d'une contention (terme ici entendu au sens d'une compression physique produite par une orthèse compressive telle que chaussette, bas ou collant) sur l'écoulement veineux de la jambe d'un sujet, et d'en mesurer les effets.

Un tel instrument, destiné à l'expérimentation clinique pratiquée par des médecins, a pour but de permettre une meilleure connaissance de l'hémodynamique des membres inférieurs, afin de mieux appréhender les effets de la compression thérapeutique de la circulation veineuse. Les mécanismes physiologiques des pathologies veineuses pourront ainsi être étudiés par acquisition de données cliniques (vitesse, débit, volume, pression, etc.) au niveau des membres inférieurs, avec et sans système de compression et, dans ce dernier cas, avec différents niveaux de compression et différents profils de compression sur la hauteur du membre.

Cette application à l'expérimentation clinique n'est cependant pas limitative, et l'on indiquera plus loin dans la description d'autres applications pour lesquelles le dispositif de l'invention peut être employé de façon particulièrement avantageuse.

Plus précisément, l'invention a pour objet de proposer un tel dispositif présentant un certain nombre de caractéristiques fonctionnelles lui permettant notamment: :
- d'appliquer une compression modulable sur tout ou partie de la jambe d'un sujet, typiquement une compression dégressive du bas vers le haut pour favoriser le retour veineux vers les parties hautes de l'organisme, et surtout une compression régulière et progressive, sans effet de garrot ni d'occlusion ;
- de ne pas faire obstacle à l'investigation des paramètres physiques et physiologiques tels que mesure des pressions ou des températures par des capteurs placés en différents points de la jambe, mesure par ultrasons de la vitesse des flux veineux (examen Doppler) ou des calibres des veines (examen échographique), ou encore mesures du volume de la jambe par pléthysmographie ;
- d'autoriser des modifications du niveau et du profil de compression sans interruption du dispositif, avantageusement par contrôle automatique informatisé ;
- de visualiser en temps réel les mesures acquises sur le sujet, pour traitement informatique ultérieur.

Jusqu'à présent, les cliniciens ont disposé de peu de dispositifs réellement efficaces pour procéder à ce type d'investigation, par example US-3 908 642, US 3 826 249 ou FR-2 616 064.

Ainsi, il a été proposé d'enfermer la jambe dans un manchon gonflable, ou dans une superposition de plusieurs manchons gonflables à des pressions différentes. Cette technique rudimentaire, si elle permet dans certains cas une compression approximative, à simple visée thérapeutique, n'est cependant pas adaptée à une étude fine des mécanismes physiologiques des pathologies veineuses.

En particulier, la pression appliquée sur le membre ne présente pas un profil régulier, comme dans le cas d'une orthèse thérapeutique où la pression que l'on cherche à reproduire est régulièrement dégressive du bas vers le haut. De plus, sur un périmètre donné, la pression n'est pas appliquée de façon homogène car le manchon, gonflé, va former des plis ayant pour conséquence une mauvaise adhérence sur la jambe et donc une application irrégulière de l'effort de compression.

L'invention, comme on l'exposera plus bas, permet de pallier l'ensemble de ces inconvénients et d'atteindre les buts recherchés avec efficacité, précision et très grande souplesse de mise en oeuvre.

À cet effet, le dispositif de l'invention comprend un manchon tubulaire entourant le membre, ce manchon comportant une partie antérieure en matériau inextensible ou relativement peu extensible, apte à venir en appui contre une région peu compressible du membre, et une partie postérieure en matériau relativement extensible, apte à envelopper la région du membre à comprimer, ces deux parties étant reliées entre elles sensiblement le long de deux génératrices du manchon tubulaire formant génératrices de liaison. Il comprend également une pluralité de ballonnets gonflables, disposés sur la face intérieure de la partie antérieure du manchon tubulaire le long d'une génératrice de celui-ci située en une position intermédiaire entre les deux génératrices de liaison des deux parties du manchon tubulaire, ces ballonnets étant aptes à venir en interposition entre le matériau inextensible et ladite région peu compressible du membre. Enfin, il est prévu des moyens pour gonfler de manière différenciée chacun des ballonnets à une pression donnée, de manière à appliquer à la partie antérieure du manchon une déformation propre à induire un effort de traction exercé sur la partie postérieure extensible, cet effort étant réparti régulièrement le long des génératrices de liaison perpendiculairement à ces génératrices, cet effort de traction entraînant lui-même, par voie de conséquence, une action de compression sur la région compressible du membre.

Selon diverses caractéristiques subsidiaires avantageuses :
- la partie antérieure comporte une fermeture à glissière s'étendant sur la majeure partie de la longueur du manchon ;
- la partie antérieure comporte, au moins sur la partie supérieure de la longueur du manchon, une fermeture autorisant en une pluralité de points un ajustement en diamètre du manchon ;
- le matériau de la partie antérieure et le matériau de la partie postérieure sont tous deux des matériaux en maille venus ensemble de tricotage ;
- le dispositif comporte en outre des capteurs de pression interposés entre le matériau de la partie postérieure et le membre à comprimer ; dans ce cas, les moyens pour gonfler de manière différenciée chacun des ballonnets peuvent avantageusement être des moyens asservis en fonction d'une comparaison opérée entre les signaux délivrés par les capteurs de pression et des valeurs de consigne correspondantes fonction d'un profil de pression recherché ;
- le dispositif comprend en outre au moins une poche gonflable disposée localement à un endroit prédéterminé de la partie postérieure du manchon tubulaire, et apte à venir combler une concavité de la région à comprimer du membre ;
- le matériau de la partie postérieure est un matériau transparent aux ultrasons.

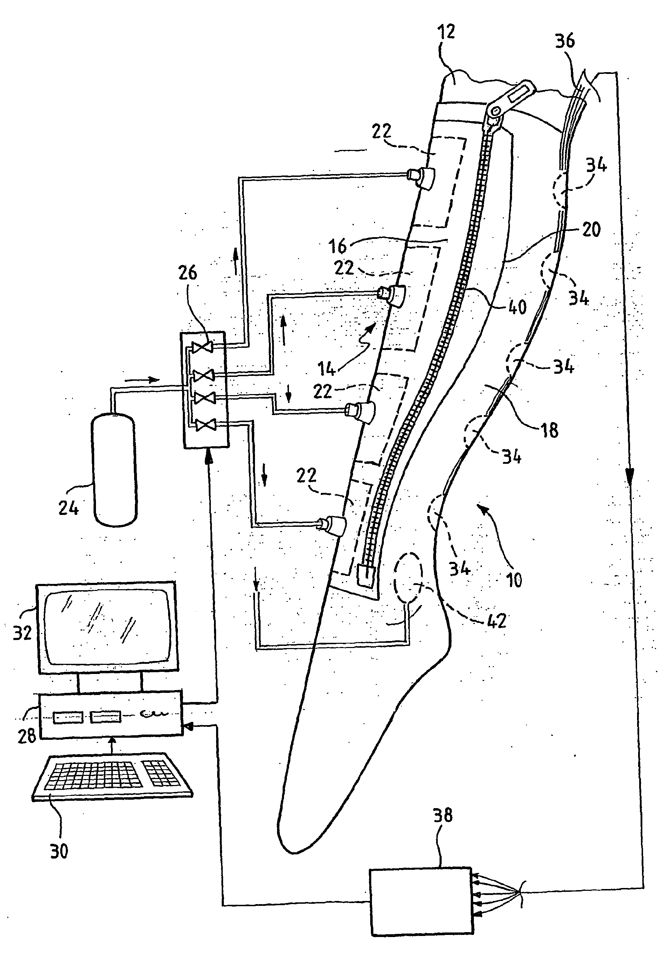

On va maintenant décrire plus en détail l'invention, en référence à la figure annexée qui illustre un mode de réalisation du dispositif de l'invention avec les moyens de commande et de mesure qui lui sont associés.

Sur la figure, la référence 10 désigne de façon générale le dispositif de l'invention, utilisé dans l'exemple illustré pour la compression de la partie de la jambe 12 comprise entre la cheville et le haut du mollet.

Le dispositif comporte essentiellement un manchon tubulaire 14 venant envelopper le mollet, en contact avec celui-ci sur toute sa surface, à la manière d'une chaussette. Ce manchon tubulaire peut être ouvert ou fermé en partie inférieure, c'est-à-dire comporter ou non un pied. Dans une variante de mise en oeuvre, il peut être prolongé vers le haut pour envelopper également le genou et la cuisse à la manière d'un bas; si l'on souhaite comprimer le membre inférieur sur toute sa hauteur.

Le manchon tubulaire 14 comprend deux parties 16,18 s'étendant chacune sur toute la longueur de la partie utile du manchon (c'est-à-dire de la région à comprimer) et raccordées sensiblement le long de deux génératrices 20 (dont une seule est visible sur la figure) situées de part et d'autre de la jambe, l'une côté intérieur, l'autre côté extérieur.

La partie 16 est une partie antérieure ou frontale, et elle s'étend le long de la crête tibiale, sur laquelle elle est approximativement centrée axialement. La partie 18, quant à elle, est une partie postérieure qui, essentiellement, enveloppe la région compressible du membre, c'est-à-dire dans le cas illustré la région du muscle du mollet. De préférence, la partie antérieure 16 présente une étendue périphérique moindre que la partie postérieure 18, c'est-à-dire que les deux génératrices 20 de raccordement de ces deux parties 16,18 se trouveront du même côté, antérieur, du membre.

La partie antérieure 16 est réalisée en un matériau essentiellement inextensible, ou rendu inextensible, mais qui soit néanmoins assez souple pour épouser la forme de la jambe. On peut utiliser par exemple un caoutchouc ou un textile faiblement élastiques ou encore un matériau tricoté à faible élasticité. L'inextensibilité de cette partie 16 peut être accrue si nécessaire par des raidisseurs appropriés, tels que des baleines introduites dans des goussets ou tout autre moyen connu analogue.

La partie postérieure 18, en revanche, est réalisée en un matériau extensible, typiquement un matériau tricoté présentant une maille du même type que celle des bas de contention classiques, par exemple les bas *Varisma* (marque déposée) d'Innothéra Topic. Cette maille peut être de type tramée, jersey, micromesh, pincée ou flottée, etc., toutes mailles connues en elles-mêmes du spécialiste de tricotage, avec un fil d'élasthanne guipé coton et polyamide, élasthanne guipé polyamide sans coton, ou encore un mélange d'élasthanne et d'élastodiène, etc.

Un tel matériau présente incidemment l'avantage d'être transparent aux ultrasons, et autorise donc toutes les investigations habituelles par mesure échographique ou Doppler sur la région du membre enveloppé par la partie postérieure 18.

Le caractère "inextensible" ou "peu extensible" de la partie antérieure 16 est entendu de manière relative, par rapport au caractère "extensible" de la partie postérieure 18. Plus précisément, lorsque l'on décrira plus bas le mode d'action du dispositif de l'invention, le matériau de la partie antérieure 16 doit être choisi, par rapport à celui de la partie postérieure 18, de manière que la pression exercée par les ballonnets 22 se traduise essentiellement par une déformation élastique de la partie postérieure 18, la partie antérieure 16 devant seulement jouer un rôle de transmission de l'effort de traction des ballonnets 22 à la génératrice 20 et, par voie de conséquence, à la partie postérieure 18. En d'autres termes, fonctionnellement, le matériau de la partie antérieure 16 doit essentiellement transmettre des efforts de traction, tandis que le matériau de la partie postérieure 18 doit essentiellement se déformer de manière élastique sous l'effet d'une traction, de manière à appliquer une pression à la partie du membre enveloppé par cette partie postérieure 18.

Les deux parties sont assemblées entre elles le long des deux génératrices 20 par tout procédé approprié tel que collage, ou soudure textile à chaud ou par haute fréquence. Avantageusement, lorsque les parties 16 et 18 présentent toutes deux une structure en maille, au lieu d'être fabriquées séparément puis réunies, elles sont réalisées simultanément par co-tricotage.

Le dispositif comporte, en outre, intercalés entre la partie antérieure 16 du manchon 14 et la crête tibiale du membre, une succession de ballonnets 22, par exemple, comme illustré, quatre ballonnets rectangulaires ayant leur grands axes alignés entre eux et alignés sur la crête tibiale. Le nombre et la forme des ballonnets ne sont cependant pas limitatifs, et dépendent du résultat recherché (plus ou moins grande finesse de réglage du profil de pression sur la hauteur du membre), de la hauteur à comprimer, etc., dès lors que, fonctionnellement, ces ballonnets permettent la mise en tension du matériau de la partie antérieure 16, comme cela sera décrit plus bas.

Ces ballonnets 22 sont reliés à une source de fluide sous pression 24, par exemple d'air comprimé, par l'intermédiaire d'une série de valves 26 commandées individuellement de manière à gonfler chacun des ballonnets à une pression donnée. Les valves 26 sont des électrovalves pilotées par un ordinateur 28 en fonction d'un programme définissant un profil de gonflage particulier. L'ordinateur 28 est en outre pourvu, de manière classique, d'un moyen d'entrée de données tel qu'un clavier 30 et d'un moyen d'affichage tel qu'un écran 32 pour la visualisation des paramètres de commande et des résultats des mesures.

Dans la région du membre que l'on souhaite comprimer, on a disposé à divers niveaux des capteurs de pression 34, interposés entre la jambe et la partie postérieure 18 du manchon 14 afin mesurer localement la pression produite. Ces capteurs sont en eux-mêmes classiques (capteurs de type Salzmann ou autres) et ne seront pas décrits plus en détail. Les signaux captés sont transmis sur des fils réunis en un faisceau 36, courant à l'intérieur du manchon tubulaire 14 puis raccordés, à l'extérieur du dispositif, à un convertisseur analogique/numérique 38 relié en entrée à l'ordinateur 28.

Pour permettre un enfilage aisé du manchon tubulaire 14 compte tenu de la variabilité des morphologies de jambe et d'une souplesse moindre que celle d'une simple chaussette en raison de la présence de la partie antérieure inextensible 16 et des ballonnets 22, on prévoit avantageusement une fermeture à glissière 40 s'étendant sur la majeure partie de la longueur du manchon, et permettant d'ouvrir le manchon 14 par exemple dans une région de la partie antérieure 16 située entre les ballonnets 22 et l'une des génératrices 20.

La partie supérieure du manchon peut être également pourvue, également sur la partie antérieure, de moyens (non illustrés) autorisant un ajustement en diamètre du manchon à plusieurs niveaux, par exemple par le biais de fermetures à bandes agrippantes, de manière à pouvoir équiper avec un même dispositif des sujets présentant des morphologies, notamment des périmètres de cheville, de mollet et/ou de cuisse variant sur des plages importantes - typiquement un périmètre de cheville compris entre 18 et 29 cm et un périmètre de mollet compris entre 25 et 45 cm).

Il est par ailleurs possible de prévoir une ou plusieurs poches gonflables 42 placées entre la jambe et le matériau de la partie postérieure 18, dans des régions telles que les régions rétromalléolaires ou sous-malléolaires. Il peut être en effet souhaitable de remplir les concavités correspondantes pour assurer une répartition plus uniforme des pressions dans la région de la cheville, par exemple dans le cas d'un pansement avec contention ajustable pour le traitement des ulcères veineux. Le rôle de ces poches gonflables 42 est toutefois entièrement différent de celui des ballonnets 20, la ou les poche(s) gonflable(s) 42 n'ayant d'ailleurs qu'un caractère subsidiaire.

On va maintenant décrire le mécanisme de mise en oeuvre de l'invention.

En gonflant de manière contrôlée les ballonnets 22, ceux-ci vont exercer un effet de traction réparti sur la partie antérieure 16, peu ou pas extensible. En effet, la partie interne de chaque ballonnet repose sur la crête tibiale, qui est une région pratiquement incompressible du membre. Comme le ballonnet est intercalé entre deux éléments inextensibles (le matériau de la partie antérieure 16 et la région tibiale du membre), son gonflage va imposer un déplacement du matériau de la partie antérieure 16. Ce déplacement à l'endroit de la crête tibiale va se transmettre, pratiquement tel quel compte tenu de l'inextensibilité du matériau, jusqu'à la génératrice 20, où il va se traduire en une force de traction exercée sur le matériau, déformable, de la partie postérieure 18. Le point d'application de cet effort de traction est situé sur la génératrice 20, donc à l'interface entre les deux parties 16 et 18, et il est orienté suivant une direction sensiblement normale à cette génératrice, c'est-à-dire une direction tangentielle au périmètre de la jambe à une hauteur donnée de celle-ci.

L'intensité de cette force va dépendre de la pression, plus ou moins grande, de gonflage des ballonnets 22 et variera graduellement sur toute la hauteur de la jambe, sans aucune irrégularité. En pratique, on constate que l'effort appliqué présente un gradient très uniforme, même avec un nombre réduit de ballonnets (par exemple quatre ballonnets seulement comme dans l'exemple illustré), et même si le profil de pression imposé par le gonflage est un profil peu conventionnel (par exemple progressif au lieu de dégressif). En particulier, le profil obtenu n'est en aucune façon un profil "en escalier" comme avec les dispositifs, rudimentaires, qui avaient pu être proposés jusqu'à présent.

Cet effort régulièrement réparti est transmis au matériau extensible de la partie postérieure 18, qui transforme cet effort en une pression de contention exercée sur la région postérieure, compressible, du membre, et ceci de façon progressive et régulière, de la même manière que ce que l'on pourrait obtenir avec un simple bas de contention.

Les capteurs 34 viennent mesurer en différents points la pression ainsi appliquée.

Avantageusement, la commande du gonflage des ballonnets 22 est asservie au signal issu de ces capteurs 34 afin de piloter les électrovannes 26 de manière à obtenir un profil de pression (mesuré par les capteurs 34) aussi proche que possible d'un profil de consigne prédéfini introduit dans le logiciel de l'ordinateur 28.

En pratique un dispositif réalisé selon les enseignements de l'invention a permis de reproduire les effets des moyens de contention physique classiques, à savoir bas ou collants de la classe I (10 à 15 mmHg, soit 13,3 à 20,0 hPa) à la classe III (20 à 36 mmHg, soit 26,6 à 47,9 hPa). De façon plus générale, un tel dispositif est capable de produire des pressions relatives comprises entre 0 et 60 mmHg (0 à 80 hPa) avec une précision de ± 1 mmHg (± 1,33 hPa).

De nombreuses applications du dispositif de l'invention peuvent être envisagées autres que l'étude expérimentale, statique, des effets d'une contention appliquée aux membres inférieurs, notamment dans le cadre de l'étude des pathologies liées à l'IVC (insuffisance veineuse chronique),

Il peut être en particulier intéressant d'enregistrer les pressions de façon dynamique, pour évaluer les variations des effets de la contention entre le repos et la marche, etc. Cette application est en effet rendue possible par le fait que le dispositif de l'invention n'implique pas d'immobilisation de la jambe dans un dispositif fixe ; au contraire, la jambe conserve toute sa liberté de mouvement, les seules contraintes étant les liaisons pneumatiques et électriques, qui peuvent être aisément conçues pour ne pas entraver les mouvements.

L'invention permet aussi d'envisager un enregistrement continu des pressions en déambulatoire sur une période longue. Tel est en particulier le cas des longs trajets en avion, où la jambe gonfle au cours du voyage et où il peut être souhaitable d'évaluer ce gonflement pour appliquer une pression de contention variable en fonction de la manière dont il évolue.

Une autre application évoquée au début de la description est celle des pansements avec contention ajustable, par exemple pour le traitement des ulcères veineux de la jambe pour lesquels une compression améliore grandement la cicatrisation et la résorption de l'ulcère, ou encore pour le traitement du lymphoedème du bras.

Le dispositif de l'invention peut être également utilisé à des fins didactiques, en faisant varier le profil de pression appliqué et en visualisant le résultat obtenu, par exemple par une mesure échographique ou Doppler.

## Revendications

1. Un dispositif pour appliquer une compression contrôlée et modulable sur un membre, **caractérisé en ce qu'**il comprend :
- un manchon tubulaire (14) entourant le membre (12), ce manchon comportant une partie antérieure (16) en matériau inextensible ou relativement peu extensible, apte à venir en appui contre une région peu compressible du membre, et une partie postérieure (18) en matériau relativement extensible, apte à envelopper la région du membre à comprimer, ces deux parties étant reliées entre elles sensiblement le long de deux génératrices du manchon tubulaire formant génératrices de liaison (20),
- une pluralité de ballonnets gonflables (22), disposés sur la face intérieure de la partie antérieure du manchon tubulaire le long d'une génératrice de celui-ci située en une position intermédiaire entre les deux génératrices de liaison des deux parties du manchon tubulaire, ces ballonnets étant aptes à venir en interposition entre le matériau inextensible et ladite région peu compressible du membre, et
- des moyens (24, 26, 28) pour gonfler de manière différenciée chacun des ballonnets à une pression donnée, de manière à appliquer à la partie antérieure du manchon une déformation propre à induire un effort de traction exercé sur la partie postérieure extensible, cet effort étant réparti régulièrement le long des génératrices de liaison perpendiculairement à ces génératrices, cet effort de traction entraînant lui-même, par voie de conséquence, une action de compression sur la région compressible du membre.

2. Le dispositif de la revendication 1, dans lequel la partie antérieure comporte une fermeture à glissière (40) s'étendant sur la majeure partie de la longueur du manchon.

3. Le dispositif de la revendication 1, dans lequel la partie antérieure comporte, au moins sur la partie supérieure de la longueur du manchon, une fermeture autorisant en une pluralité de points un ajustement en diamètre du manchon.

4. Le dispositif de la revendication 1, dans lequel le matériau de la partie antérieure et le matériau de la partie postérieure sont tous deux des matériaux en maille venus ensemble de tricotage.

5. Le dispositif de la revendication 1, comportant en outre des capteurs de pression (34) interposés entre le matériau de la partie postérieure et le membre à comprimer.

6. Le dispositif de la revendication 5, dans lequel les moyens pour gonfler de manière différenciée chacun des ballonnets sont des moyens asservis en fonction d'une comparaison opérée entre les signaux délivrés par les capteurs de pression et des valeurs de consigne correspondantes fonction d'un profil de pression recherché.

7. Le dispositif de la revendication 1, comprenant en outre au moins une poche gonflable (42) disposée localement à un endroit prédéterminé de la partie postérieure du manchon tubulaire, et apte à venir combler une concavité de la région à comprimer du membre.

8. Le dispositif de la revendication 1, dans lequel le matériau de la partie postérieure est un matériau transparent aux ultrasons.

## Claims

1. A device for applying a controlled and modular compression onto a limb, **characterized in that** it comprises:
- a tubular sleeve (15) surrounding the limb (12), said sleeve comprising an anterior part (16) of an inextensible or relatively not very extensible material, adapted to bear against a not very compressible region of the limb, and a posterior part (18) of a relatively extensible material, adapted to envelop the region of the limb to be compressed, said two parts being connected essentially along two forming generatrices of the tubular sleeve as connecting generatrices (20),
- a plurality of inflatable balloons (22) disposed on the inner side of the tubular sleeve along a generatrix of the latter, situated in an intermediary position between the two connecting generatrices of the two parts of the tubular sleeve, wherein said balloons are adapted to come between the inextensible material and said not very compressible region of the sleeve, and
- means (24, 26, 28) adapted to differentially inflate each of the balloons to a given pressure so as to apply to the anterior part of the sleeve a deformation that is able to induce a tensile stress exerted onto the posterior extensible part, said stress being regularly distributed along the connecting generatrices perpendiculary to said generatrices, wherein as a consequence said tensile stress results to a compression action onto the compressible region of the limb.

2. The device according to claim 1, wherein the anterior part comprises a zip fastener (40) extending oh the major part of sleeve's length.

3. The device according to claim 1, wherein the anterior part comprises, at least on the posterior part of sleeve's length, a fastener allowing on a plurality of points an adjustment of sleeve's diameter.

4. The device according to claim 1, wherein the material of the anterior part and the material of the posterior part are both knitted mesh materials.

5. The device according to claim 1, furthermore comprising pressure transducers (34) disposed between the material of the posterior part and the limb to be compressed.

6. The device according to claim 5, wherein the means adapted to differentially inflate each of the balloons are controlled as a function of a comparison made between signals delivered by the pressure transducers and corresponding set values depending on a wanted pressure profile.

7. The device according to claim 1, furthermore comprising at least one inflatable pouch (42) locally disposed on a predetermined place of the posterior part of the tubular sleeve and adapted to fill a concavity of the region of the limb to be compressed.

8. The device according claim 1, wherein the material of the posterior part is an ultrasound permeable material.

## Patentansprüche

1. Eine Einrichtung zum Anlegen eines kontrollierbaren und modulierbaren Drucks auf ein Glied, **dadurch gekennzeichnet, dass** sie
- eine das Glied (12) umgebendes, ein vorderes Teil aus einem undehnbaren oder vergleichsweise wenig dehnbaren Material aufweisende rohrförmige Manschette, die geeignet ist, mit einem wenig dehnbaren Bereich des Glieds in Anlage zu kommen, und ein hinteres Teil 18 aus einem vergleichsweise dehnbaren Material, das geeignet ist, den Bereich des zu komprimierenden Glieds einzuhüllen, wobei diese zwei Teile miteinander im Wesentlichen entlang der zwei Verbindungserzeugenden (20) bildenden Erzeugenden der rohrförmigen Manschette verbunden sind,
- eine Vielzahl von aufblasbaren Ballonen (22), die sich auf der inneren Seite des vorderen Teils der rohrförmigen Manschette entlang deren in einer zwischenliegenden Lage zwischen genannten Verbindungserzeugenden der zwei Teile der rohrförmigen Manschette liegender Erzeugenden befinden, wobei diese Ballone geeignet sind, zwischen dem undehnbaren Material und dem wenig kompressierbaren Bereich des Glieds eingefügt zu werden,
- Mittel (24,216,28) zum differenziellen Aufblasen jedes der Ballone auf einen vorgegebenen Druck, um an das vordere Teil der Manschette eine auf das dehnbare hintere Teil ausgeübten Zugspannung herbeizuführenden Verformung anzulegen, wobei diese Spannung regelmässig entlang der Verbindungserzeugenden senkrecht auf diesen Erzeugenden verteilt ist, wobei demzufolge diese Zugspannung selbst eine Druckaktion auf das komprimierbare Teil des Glieds ausübt,
umfasst.

2. Die Einrichtung gemäss Anspruch 1, in welcher das vordere Teil einen sich auf dem grössten Teil der Länge der Manschette erstreckenden Reissverschluss aufweist.

3. Die Einrichtung gemäss Anspruch A, in welcher das vordere Teil auf wenigstens dem oberen Teil der Länge der Manschette einen an mehreren Punkten eine Einstellung des Durchmessers der Manschette erlaubenden Verschluss aufweist.

4. Die Einrichtung gemäss Anspruch 1, in welcher beide Materiale des vorderen Teils und des hinteren Teils beide gestrickte Maschenmateriale sind.

5. Die Einrichtung gemäss Anspruch 1, umfassend ausserdem zwischen dem Material des hinteren Teils und dem zu komprimierenden Glied zwischengelagerte Drucksensoren (34) .

6. Die Einrichtung gemäss Anspruch 5, in welcher die Mittel zum differenziellen Aufblasen jedes der Ballone Regelungsmittel sind, die von einem Vergleich zwischen den durch die Drucksensoren gelieferten Signalen und den entsprechenden, von einem gesuchten Druckprofil abhängigen Sollwerten abhängig sind.

7. Die Einrichtung gemäss Anspruch 1, umfassend ausserdem eine aufblasbare, örtlich auf ein vorgegebenes Ort des hinteren Teils der rohrförmigen Manschette befindliche Tasche (42), die geeignet ist, eine Konkavität des zu komprimierenden Bereichs des Glieds aufzufüllen.

8. Die Einrichtung gemäss Anspruch 1, in welcher das Material des hinteren Teils ein ultraschalldurchlässiges Material ist.
